Europäisches Patentamt

(19) European Patent Office

Office européen des brevets

(11) Publication number: **0 064 275**
**B1**

(12) **EUROPEAN PATENT SPECIFICATION**

(45) Date of publication of patent specification: **21.08.85**

(21) Application number: **82103640.7**

(22) Date of filing: **28.04.82**

(51) Int. Cl.⁴: **G 01 N 33/53** // G01N33:546

(54) **Immunochemical reagent.**

(30) Priority: **02.05.81 JP 67332/81**

(43) Date of publication of application:
**10.11.82 Bulletin 82/45**

(45) Publication of the grant of the patent:
**21.08.85 Bulletin 85/34**

(84) Designated Contracting States:
**AT BE CH DE FR GB IT LI NL SE**

(56) References cited:
**GB-A-1 346 862**
**GB-A-2 013 211**
**US-A-3 912 805**
**US-A-4 092 114**
**US-A-4 118 192**

(73) Proprietor: **MITSUBISHI CHEMICAL
INDUSTRIES LIMITED**
**5-2, Marunouchi 2-chome Chiyoda-ku
Tokyo 100 (JP)**

(72) Inventor: **Tsutsui, Satoshi**
**13-13, Tsukimino 7-chome
Yamato-shi Kanagawa (JP)**
Inventor: **Sudo, Tadamitsu**
**13-3, Misono 1-chome Sagamihara-shi
Kanagawa (JP)**
Inventor: **Ito, Michio**
**9-107, Wakabadai 1-chome Asahi-ku
Yokohama-shi Kanagawa (JP)**

(74) Representative: **Patentanwälte TER MEER -
MÜLLER - STEINMEISTER**
**Mauerkircherstrasse 45**
**D-8000 München 80 (DE)**

Courier Press, Leamington Spa, England.

## Description

1. Field of the invention.

This invention relates to a sensitized particulate reagent for immunological reactions.

2. Description of the prior art.

In recent years it has become a very important subject in various fields such as medicine, biochemistry, hygiene and epidemiology to assay trace substances, particularly antigens and antibodies.

The prior art methods heretofore employed widely for this purpose comprise reacting an antigen or antibody with latex particles sensitized with the appropriate antibody or antigen on a glass plate and visually observing the degree of agglutination (for example GB—A—1 346 862, US—A—3 912 805 and US—A—4 092 114). With these methods however, it is difficult to perform the assay with high accuracy.

It has recently been proposed as a modification of the above prior art assay system for antigens and antibodies using sensitized latex particles that the rate of decrease in turbidity of the supernatant of the latex after the reaction can be optically measured to perform the assay utilizing the phenomenon that the reaction of an antigen- or antibody-sensitized latex with an antibody or antigen causes agglutination of the latex particles (for example *Croatia Chemica Acta,* Vol. 42, p.p. 457—466), Yugoslavia; *European Journal of Biochemistry*, Vol. 20, No. 4, p.p. 558—560 (1971), Germany; *Immunochemistry*, Vol. 12, p.p. 349—351 (1975), U.K. and UK—A—2 013,211). However, even such modified methods fail to afford satisfactory results in terms of accuracy and reproducibility and have not yet been put into practice.

It has also been proposed that the assay is carried out with light of a specific wavelength using carrier particles having a specific particle diameter (e.g. US—A—4 118 192).

However, the prior art reagents for the assay of an antigen or antibody with high sensitivity which comprises fine particles sensitized with appropriate antibody or antigen involve in common the disadvantages that the dispersibility of the sensitized particles may so be deteriorated that they are susceptible to non-specific agglutination reactions and that their sensitivity and immunoreactivity may vary greatly during storage, resulting in significant damage to detection and accuracy.

Summary of the invention

The inventors have investigated with the intention of developing sensitized particulate reagents for immunological reactions which are free of the abovementioned disadvantages and found that the medium in which the sensitized particles are dispersed is critical, thereby accomplishing this invention.

The present invention therefore comprises a sensitized particulate reagent for immunological reactions, which is characterized by comprising particulate carriers having an average particle diameter of 0.05 to 1.2 μm suspended at a concentration of 0.01 to 20% in an aqueous medium having an electric conductivity of 5.0 mS/cm (5.0 mΩ$^{-1}$·cm$^{-1}$) or less and a pH of from 5 to 10, said particulate carriers being sensitized with an antigen or antibody.

Detailed description of the preferred embodiments

Now the present invention is described in detail.

The particulate carrier which is a constituent of the reagent of this invention comprises usually such particles that are substantially insoluble in aqueous media. Such particulate carriers may be made of any suitable material which includes latices of polymeric materials such as polystyrene, styrene-butadiene copolymer, polyacrylate, polymethacrylate, acrylonitrile-butadiene-styrene copolymer, latices of these polymers which have been activated by introducing a carboxyl or amide group by means of copolymerization with acrylic acid, methacrylic acid and acrylamide; blood cells, bacteria such as staphylococci and streptococci, Serratia marcescens or rickettsia and fragments of these bacteria.

The average paricle diameters of these carriers are from 0.05 to 1.2 μm, preferably from 0.1 to 1.0 μm and more preferably from 0.2 to 0.8 μm. If the diameter of the particulate carrier is much too large, the range of assay is restricted or becomes more unstable. On the other hand, particulate carriers having much smaller diameter bring about a great economical loss in the preparation of the reagent and cannot easily bring about an improved sensitivity. Therefore, neither such an excessively large nor small particle diameter is preferred.

The antigens to be supported on the particulate carrier of the present invention reagent include, for example, proteins, polypeptides, steroids, polysaccharides, lipids, pollens, dusts and haptens. The antibodies include, for example, those proteins produced by reaction with the above-mentioned antigens.

The concentration of the particulate carrier is usually 0.03% to 1.09%, preferably 0.05% to 0.5% as final concentration. At a higher concentration of the particulate carrier, it is necessary to use a more complicated equipment for the assay with the reagent of this invention while any further improvement in effect is not attained. On the other hand, a lower concentration is also not preferred because it fails to give satisfactory sensitivity and reactivity.

The particulate carrier may be sensitized with an antigen or antibody by any technique known per se, i.e., by physical adsorption of the antigen or antibody on the particulate carrier, by chemical bonding between the antigen or antibody and the particulate carrier or by combination of both.

The carrier is sensitized with the antigen or antibody the amount of which may be suitably selected depending on various factors including the type of the particular antigen or antibody and the intended accuracy of assay with the reagent.

The particulate carrier sensitized with an antigen or antibody may be treated with a stabilizer before it is suspended in an aqueous medium. The stabilizer useful for this purpose includes amino acids, polypeptides and proteins which do not participate in the intended immunological reaction and serum albumin is most preferred as a stabilizer. The treatment with the stabilizer may be carried out in a manner known per se and such treatment is advantageous from the viewpoint of storage and stability of the reaction. Particularly significant effect is attained in the cases where the antigen or antibody is physically adsorbed on the carrier.

The aqueous medium in which the carrier sensitized with the antigen or antibody is suspended must have an electric conductivity of 5.0 mS/cm or less, preferably 2.5 mS/cm or less and most preferably 1.0 mS/cm or less. An electric conductivity higher than 5.0 mS/cm makes the reagent unstable, resulting in a decreased accuracy of assay.

The aqueous medium may be water or a buffer solution, which may contain one or more additives selected from stabilizers, preservatives, chelating agents and surfactants. The buffer solution includes glycine buffers, phosphoric acid buffers, citric acid buffers, barbital buffers, borate buffers, Tris[tris(hydroxymethyl)-aminomethane]-hydrochloric acid buffers, Tris-malate buffers and ammonia buffers.

The stabilizers includes, for example, the aforementioned ones and they are usually present at concentrations of 0.001% to 1%, preferably 0.05% to 0.6%.

Preferred examples of the preservatives include sodium azide and merthiolate.

Preferred examples of the chelating agents include ethylenediaminetetraacetic acid, nitrilotriacetic acid and cyclohexanediaminetetraacetic acid.

As the surfactant, nonionic surfactants are generally preferred.

The pH of the aqueous medium is from 5 to 10, preferably from 6 to 9.5 and most preferably 6.5 to 8.5. If the pH of the aqueous medium is lower than 5, the reagent may become unstable, although the sensitivity is increased. An aqueous medium having a pH value exceeding 10 is also undesired because it results in a decreased sensitivity and in some instances an unstable storage of the reagent.

The particulate carrier sensitized with an antigen or antibody is suspended in the aqueous medium at a concentration of 0.01% to 20%, preferably 0.05% to 10%. At a lower concentration, the resulting reagent has a decreased stability and its usage becomes inconvenient and restricted, while at a higher concentration the stability is also decreased and such concentration is inconvenient and not preferred.

The reagent of this invention may be prepared, for example, in a manner as described in the Examples given below, i.e., by sensitizing particulate carriers as described above with an antigen or antibody in a manner known per se, then, if necessary, treating the sensitized carrier with a stabilizer and suspending the carrier in an aqueous medium as described above in a manner known per se.

The reagent according to this invention is suitable as a reagent or a stock solution thereof for use, for example, in a method of reacting on a slide glass or in a test tube and visually observing the agglutination or in a method of reacting in an optical cell and optically measuring the reaction.

The reagent may be used to assay an immunological reaction in a manner mentioned below.

First, if necessary, the reagent according to this invention is diluted with a buffer solution to adjust the concentration of the carrier sensitized with an antigen or antibody so that it is suitable for the assay of the immunological reaction. The suitable concentration depends on various factors including the particular antigen or antibody to be assayed, its concentration and reactivity and can be determined experimentally. In general, in the case of optical measurement of the immunological reaction, the final concentration of the carrier be at least 0.03%, preferably from 0.05% to 1% and more preferably 0.1% to 0.5% by weight. In the case of visual observation of the immunological reaction, the final concentration is in the range of 0.05% to 0.8%, preferably 0.1% to 0.5% by weight.

The buffer solutions which can be used to dilute the reagent include glycine buffers, borate buffers, Tris-hydrochloric acid buffers, Tris-malate buffers and ammonia buffers.

The pH of the buffer solution used is usually in the range of 7.6 to 9.6, preferably in the range of 8.0 to 9.0. A buffer solution of lower pH value tends to cause agglutination, thereby making the reagent unstable, although it results in an increased sensitivity. At a higher pH, the sensitivity is decreased and storage properties at elevated temperatures may undesirably deteriorate.

The thus obtained reagent can be used to measure an immunological reaction in a conventional manner.

Thus, in the cases where the immunological reaction is observed visually, predetermined amounts of the reagent and an antigen- or antibody-containing sample are mixed on a slide glass or in a test tube and the agglutination of the sensitized particles is observed.

In the cases where the immunological reaction is measured optically, predetermined amounts of the reagent and an antigen- or antibody-containing sample are mixed and the change in light transmittance, scattered light or turbidity is determined in an optical cell.

When the optical measurement is carried out by determination of light transmittance, the wavelength of the light is usually greater than the average particle diameter of the carrier by a factor of at least 1.1, preferably at least 1.5 and more preferably at least 2. The wavelength is usually in the range of 600 to 2,400 nm, preferably 800 to 1,800 nm. If the light is of lower wavelength, it is necessary in order to obtain an appropriate light transmittance to use a particulate carrier having sufficiently low concentration along with a reagent having an extremely good dispersibility and therefore it is difficult to enhance the sensitivity and in some instances reversal of the reaction may occur. A longer wavelength is also undesirable because the sensitivity is decreased.

The light may be either monochromatic or polychromatic.

The optical cell used in the measurement of light transmittance usually has a thickness of 0.2 to 10 mm.

The measurement of transmittance may be effected by determining the time taken to reach a predetermined value of absorbance, or by determining the increase in absorbance in a predetermined length of time.

The optical measurement by use of scattered light may be effected in the same way as in the above cases where light transmittance is determined.

The reagents according to this invention are sensitized particulate reagents for use in immunological reactions which can determine the amounts of an antigen or antibody in various samples with good reproducibility and high accuracy. In addition, they are effective after they have been frozen and re-melted and they can therefore be lyophilized and reused.

Having generally described the invention, a more complete understanding can be obtained by reference to certain specific examples, which are included for purposes of illustration only and are not intended to be limiting unless otherwise specified.

Example 1

Antibody globulin against α-fetoprotein was dissolved in 0.1 M glycine buffer (pH 8.8). The number of the antibody globulins was equivalent to 800 to 1,500 for each latex particle (of 0.220 μm diameter, manufactured by Dow Chemical). The solution was then mixed with an equal volume of latex-glycine buffer (pH 8.8) at room temperature under stirring to effect the sensitization adequately. After centrifugal separation, the supernatant was removed and the precipitate was treated with an appropriate amount of bovine serum albumin. The thus obtained sensitized particles were suspended in a buffer solution having an electric conductivity of 1.5 mS/cm and containing an appropriate amount of a preservative and the suspension was stored. The resulting reagent showed an extremely good accuracy in assay.

The reagent was diluted and used to assay an immunological reaction.

The dilution was conducted with a glycine buffer (pH 8.6). The results of the assay in terms of within-run precision and day-to-day precision are summarized in Tables 1 and 2.

TABLE 1
Within-run precision

| No. | Serum sample ng/ml | | |
| --- | --- | --- | --- |
| | 1 (45.0)* | 2 (195.6)* | 3 (950.0)* |
| 1 | 45.2 | 189.2 | 906.2 |
| 2 | 41.3 | 190.5 | 917.5 |
| 3 | 42.0 | 195.8 | 887.5 |
| 4 | 44.5 | 181.1 | 901.3 |
| 5 | 44.6 | 190.5 | 906.3 |
| 6 | 43.6 | 189.7 | 937.1 |
| 7 | 42.5 | 190.1 | 885.0 |
| 8 | 42.0 | 184.5 | 918.0 |
| 9 | 38.9 | 180.5 | 913.5 |
| 10 | 38.6 | 184.2 | 925.3 |
| 11 | 40.2 | 185.6 | 921.0 |
| 12 | 39.7 | 190.1 | 887.2 |
| Average | 41.9 | 187.7 | 908.8 |
| Standard deviation | 2.26 | 4.49 | 16.4 |
| Coefficient of variation | 5.4% | 2.4% | 1.8% |

*The values in parentheses are those determined by radioimmunoassay.

TABLE 2
Day-to-day precision

| No. | Month/ Day (1980) | Serum sample ng/ml | | |
| --- | --- | --- | --- | --- |
| | | 1 | 2 | 3 |
| 1 | 2/4 | 55.3 | 153.0 | 530.6 |
| 2 | 2/7 | 49.2 | 145.8 | 517.6 |
| 3 | 3/5 | 54.3 | 154.1 | 542.9 |
| 4 | 3/27 | 45.5 | 149.0 | 464.3 |
| 5 | 4/10 | 47.5 | 146.0 | 483.3 |
| 6 | 5/7 | 48.5 | 151.0 | 500.8 |
| 7 | 5/14 | 47.8 | 148.3 | 478.3 |
| 8 | 5/21 | 51.8 | 138.2 | 507.3 |
| 9 | 6/10 | 50.3 | 159.7 | 512.8 |
| 10 | 6/25 | 52.1 | 156.3 | 521.3 |
| Average | | 50.2 | 150.1 | 505.9 |
| Standard deviation | | 3.13 | 6.12 | 24.5 |
| Coefficient of variation (%) | | 6.2 | 4.1 | 4.9 |

Example 2

Anti-CRP (C-reactive protein) antibody was dissolved in 0.2 M borate buffer (pH 8.8) and latex particles (of 0.220 μm diameter, manufactured by Dow Chemical) suspended in the same buffer were so sensitized with the resulting solution that each particle carried 800 to 1,800 antigens. The sensitized latex particles were treated with bovine serum albumin and dispersed in an appropriate amount of borate buffer (4.0 mS/cm). The resulting reagent showed an extremely good accuracy of measurement.

Example 3

Rabbit or goat anti-human fibrinogen antibody F (ab')₂ was dissolved in 0.1 M Tris-malate buffer (pH 8.6) and latex particles (of 0.220 μm diameter, manufactured by Dow Chemical) suspended in the same buffer were so sensitized with the resulting solution that the sensitivity of the resulting reagent was on the order of 100 ng/ml. The particles were then treated with bovine serum albumin and suspended in an aqueous solution containing 0.1% sodium azide (1.0 mS/cm). In use, the reagent is diluted with a Tris-malate buffer (pH 8.4) or Tris-hydrochloric acid buffer (pH 8.4) to a desired latex concentration and reacted on a slide glass, or it can serve as a reagent for optical assay as it is without dilution. In a reaction on a slide glass using this reagent, the aggulutination pattern and non-agglutination pattern can be distinguished clearly.

Example 4

Goat antibody globulin against human IgG is dissolved in 0.1 M Tris-malate buffer (pH 8.8) and latex particles of 0.312 μm diameter, manufactured by Dow Chemical) suspended in the same buffer is sensitized with the solution. The sensitized particles are then treated with bovine serum albumin and finally suspended in an aqueous solution having a conductivity of about 3.0 mS/cm. In use, the suspension is diluted with 0.1 M Tris-malate or Tris-HCl buffer to a desired concentration or used as it is.

Example 5

Rabbit anti-human IgA antibody F (ab')₂ is dissolved in 0.2 M borate buffer (pH 8.3) and the solution is treated in the same manner as described in Example 4 to prepare a desired reagent in suspension except that the aqueous solution used to finally suspend the sensitized particles has a conductivity of about 0.5 to 0.8 mS/cm.

Example 6

Latex particles are sensitized with highly purified rabbit anti-human IgM antibody in a glycine buffer of pH 9.6. The quantitative relationship varies depending on the quality of the antibody and the desired sensitivity. After the sensitized particles are treated with an appropriate amount of bovine serum albumin, they are suspended in an aqueous solution having a conductivity of about 1.2 mS/cm (which may contain approximately 0.05% by bovine serum albumin) to give a desired reagent.

Example 7

Highly purified rabbit anti-hCG antibody F (ab')₂ is dissolved in 0.2 M Tris-HCl buffer (pH 8.6) and the resulting solution is used to sensitize therewith latex particles (of 0.220 μm diameter, manufactured by Dow Chemical) suspended in the same buffer at room temperature. After treatment with an appropriate amount of bovine serum albumin, the sensitized particles are suspended in an aqueous solution having a conductivity of about 0.8 mS/cm and stored.

Claims

1. A sensitized particulate reagent for immunological reactions, characterized by comprising particulate carriers having an average particulate diameter of 0.05 to 1.2 μm suspended at a concentration of 0.01 to 20% in an aqueous medium having an electric conductivity of 5.0 mS/cm or less and a pH of from 5 to 10, said particulate carriers being sensitized with an antigen or antibody.

2. The reagent as defined in claim 1, characterized in that the aqueous medium has an electric conductivity of 2.5 mS/cm or less.

**Patentansprüche**

1. Sensibilisiertes teilchenförmiges Reagens für immunologische Reaktionen, dadurch gekennzeichnet, daß es Trägerteilchen mit einem durchschnittlichen Teilchendurchmesser von 0.05 bis 1,2 μm enthält, die in einer Konzentration von 0,01 bis 20% in einem wäßrigen Medium mit einer elektrischen Leitfähigkeit von 5,0 mS/cm oder weniger und einem pH-Wert von 5 bis 10 suspendiert sind, welche Trägerteilchen mit einem Antigen oder Antikörper sensiblisiert sind.

2. Reagens nach Anspruch 1, dadurch gekennzeichnet, daß das wäßrige Medium eine elektrische Leitfähigkeit von 2,5 mS/cm oder weniger aufweist.

**Revendications**

1. Réactif particulaire sensiblisé pour des réactions immunologiques, caractérisé en ce qu'il comprend des supports particulaires ayant un diamètre particulaire moyen de 0,05 à 1,2 μ en suspension en une concentration de 0,01 à 20% dans un milieu aqueux ayant une conductivité électrique de 5,0 mS/cm ou moins et un pH de 5 à 10, ces supports particulaires étant sensibiliés avec un antigène ou un anticorps.

2. Réactif suivant la revendication 1, caractérisé en ce que le milieu aqueux a une conductivité électrique de 2,5 mS/cm ou moins.